# EUROPEAN PATENT SPECIFICATION

(11) **EP 1 091 710 B1**
(45) Date of publication and mention of the grant of the patent: **27.11.2002**
(21) Application number: 99925239.8
(22) Date of filing: 24.06.1999
(51) Int. Cl.: A61F 13/15

(54) **METHOD AND DEVICE FOR TRANSFORMING A TWO-DIMENSIONAL SANITARY NAPKIN INTO A THREE-DIMENSIONAL ONE**
VERFAHREN UND VORRICHTUNG ZUR UMFORMUNG EINER ZWEIDIMENSIONALEN DAMENBINDE IN EINE DREIDIMENSIONALE DAMENBINDE
PROCEDE ET DISPOSITIF DE TRANSFORMATION D'UNE SERVIETTE HYGIENIQUE BIDIMENSIONNELLE EN UNE SERVIETTE HYGIENIQUE TRIDIMENSIONNELLE

(30) Priority: 30.06.1998 US 107375
(43) Date of publication of application: 18.04.2001
(73) Proprietor: The Procter & Gamble Company, Cincinnati, Ohio 45202 (US)
(72) Inventor: AOUAD, Yousef, Cincinnati, OH 45213 (US); BIEN, Denise, Jean, Cincinnati, OH 45208 (US); ALVIS, Cynthia, Lee, Fairfield, OH 45014 (US)
(74) Representative: Hirsch, Uwe Thomas
(86) International application number: IB9901187
(87) International publication number: WO00000121

(56) References cited:
- EP-A- 0 651 986
- WO-A-95/17150
- DE-C- 564 905
- US-A- 5 127 911

## Description

### TECHNICAL FIELD

This invention discloses a substantially flat sanitary napkin, i.e., one positioned almost exclusively in the x-y plane, having an expandable member therein that once activated by a female wearer either densifies in a prescribed zone and/or expands at least a portion of the sanitary napkin out of the x-y plane and into the z-plane to thereby create hump. Such activation occurs when cinch members are used to contract the expandable member in the x and/or y-directions while simultaneously causing its densification or expansion into the z-plane.

### BACKGROUND OF THE INVENTION

All manner and variety of absorbent devices or appliances have been configured for the absorption of body fluids, such as menses, and are well known. Such devices are expected to absorb the body fluid, retain the fluid within the absorbent and to prevent the discharged body fluids from soiling the person and/or clothing.

In the formation of such disposable absorbent devices they commonly include a liquid-permeable, bodyfacing cover, an absorbent core and a liquid-impermeable backing sheet or baffle. These absorbent devices, whether utilized as diapers, incontinence garments or sanitary napkins are subject to failure. Leakage from absorbent devices is generally attributed to a high concentration of fluid absorption at the point of fluid insult. At this point the absorbent material in the device becomes super-saturated and unable to accept, to a large degree, additional fluids from the body. Using a sanitary napkin as an example, the menses will generally migrate radially from the point of insult and will leak from the sides. This usually results in the soiling of a female wearer, typically around her thigh region, and her undergarment. In the area of sanitary napkins, it has been suggested that at least 20-25 percent of some sanitary napkins experience side leakage. This incidence of leakage increases for those sanitary napkins having increased absorbency designed primarily for medium to heavy flow.

To overcome the problem of side leakage, sanitary napkins have been constructed having elasticized sides that urge the sides upward or cause the sanitary napkin to form a cup shape.

Another method of preventing side leakage has been to extend wings, flaps or panels (hereinafter wings) from the edges of the sanitary napkin. The wings generally extend over the edges of the undergarment and adhere to the underside of the crotch portion or to themselves. The wings typically assist the garment adhesive, if present, to hold the sanitary napkin in position during use.

However, it is possible that these elasticized edges or wings will fold inward, partially occluding the cover surface and thereby diminishing the efficacy of the sanitary napkin. In some cases this folding results in the edges actually contributing to the incidence of failure.

Improving the performance of sanitary napkins continues to be a formidable undertaking, although a number of improvements have been made in both materials and construction. However, eliminating leakage, particularly along the inside of the thighs without compromising comfort and fit has not met the desired needs of the consumer.

Therefore, there remains a need for a sanitary napkin that will be comfortable to wear while decreasing the chance of side leakage associated with the use of sanitary napkins during the menstrual period.

In response to consumers' needs, a number of devices have arisen.

For example, U.S. Patent No. 5,300,055 entitled Absorbent Article Having A Thermoplastic Deformable Element issued on April 5, 1994 is directed to a disposable absorbent article, and particularly a sanitary napkin, having a flexure-resistant deformation element, the deformation element having a body facing surface which has a convex upward configuration when the sanitary napkin is worn.

Also, U.S. Patent No. 5,688,259 entitled Absorbent Article Having A Resilient Center issued on November 18, 1997 describes an absorbent article, such as a sanitary napkin that has a body-conforming configuration provided by a longitudinally-oriented resilient hump-forming element. In particular, the hump-forming element is positioned to form a hump along the longitudinal centerline on the body surface of the sanitary napkin.

Additionally, U.S. Patent No. 5,545,156 entitled Absorbent Article Having A Preformed Member issued on August 13, 1996 describes an absorbent article with an outer perimeter, a cover, a liquid-impermeable preformed member and an absorbent core positioned therebetween. The pre-formed member has a bulge therein that provides close body contact between the wearer and the absorbent article. The bulge extends above the bodyfacing surface of the absorbent core.

Lastly, U.S. Patent No. 5,624,423 entitled Absorbent Article Having A Barrier Means And A Medial Bulge issued on April 29, 1997 provides an absorbent article with an outer perimeter having a cover, a baffle, an absorbent core and a barrier means for intercepting fluid migrating from the absorbent core toward the outer perimeter of the article. The absorbent core has a protuberance that extends above a plane that is parallel to the absorbent core periphery.

All of the above cited prior art is at least alike in one respect: each discloses an absorbent article that provides a pre-formed bulge or hump that breaks the x-y plane of the absorbent article, such bulge or hump residing substantially in the z-plane.

When such an absorbent article is formed, new issues of packaging are created. Also, due to the pre-formed nature of the articles, there can be no variation in the height of the bulge or its fit within the perineal grooves of a female wearer.

As a solution to this, it is the object of the invention heiein to provide a sanitary napkin having a mechanically expandable element therein, such element being expandable by a user before and/or during use of the sanitary napkin.

It is a further object herein that the mechanically expandable element in the sanitary napkin be variable by a user such that the right fit and impact of the element with a user's perineum is achieved.

It is an additional object of the sanitary napkin herein that the napkin is able to be packaged like a traditional thin, flat sanitary napkin without requiring the increased packaging of a pre-formed hump sanitary napkin.

### SUMMARY OF THE INVENTION

Accordingly, the present invention provides a sanitary napkin having a center, a topsheet, a backsheet attached to the topsheet, an absorbent core positioned between the topsheet and the backsheet, an expandable zone, an expandable member located within the expandable zone and positioned substantially about the x-axis having a first end and a second end positioned between the topsheet and the backsheet, and at least a pair of cinch members, one cinch member being attached at the first end of the pre-folded, expandable member, the other cinch member being attached to the second end of the expandable member, and preferably a confining channel housing the expandable member and the cinch members. The channel confines the relative motion of the expandable member and the cinch members in a prescribed direction; namely, in the x-plane.

In the preferred embodiment herein, each cinch member extends out between the topsheet and the backsheet such that when the cinch members are pulled oppositely to one-another, the ends of the expandable member are pulled together to form a densified zone in the sanitary napkin. As many zones of densification as desired may be formed within one sanitary napkin.

In another embodiment herein, each cinch member extends out between the topsheet and the backsheet such that when the cinch members are pulled oppositely to one-another, the ends of the expandable member are pulled toward the center of the expandable member to form a raised and puffed hump that protrudes substantially into the z-plane.

The expandable member may be raised at various heights; e.g., from about 3 mm to about 50 mm. Furthermore, the expandable member, by virtue of its composition may be as stiff or as pliable as determined by a manufacturer. Such a determination of stiffness or pliability will depend upon materials used in the expandable member, as well as the number of layers, height of the hump, contour of the hump, shape of the hump, position of the hump along the upward surface of the sanitary napkin and whether it is above or below the topsheet of the sanitary napkin. Preferably, once the expandable member has been activated to form a hump by a user to a specific position, it will substantially hold that position throughout the use of the sanitary napkin. Also preferably, at least one expandable zone of the sanitary napkin is positioned to be within the zone of menses insult by a female user.

The expandable member of the pad may comprise crease lines. These crease lines are normally oriented from one side of the confining channel and extend to one longitudinal edge of the expandable member. The crease lines may be formed between the top layer and bottom layer of the expandable member by a number of known bonding processes in the art such as thermal bonding, adhesive bonding and/or mechanical bonding.

### BRIEF DESCRIPTION OF THE DRAWINGS

While the specification concludes with claims particularly pointing out and distinctly claiming the subject matter which is regarded as forming the present invention, it is believed that the invention will be better understood from the following descriptions which are taken in conjunction with the accompanying drawings in which like designations are used to designate substantially identical elements, and in which:
Figure 1 is a perspective view of a sanitary napkin with a hump formed therein;
Figure 1A is a plan view of the mechanically expandable sanitary napkin laid out in the x-y plane in its pre-expanded configuration;
Figure 2 is an exploded view of the expandable member of the sanitary napkin showing the expandable member and cinch members;
Figure 3 is an exploded view of an alternative embodiment of the expandable member of the sanitary napkin showing the expandable member and cinch members;
Figure 4 is an exploded view of an alternative embodiment of the expandable member of the sanitary napkin showing the expandable member and cinch members;
Figure 5 is a cross-sectional view of the expandable member combined with a top view of a cinch profile;
Figure 6 is a cross-sectional view of an alternative embodiment of the expandable member; and

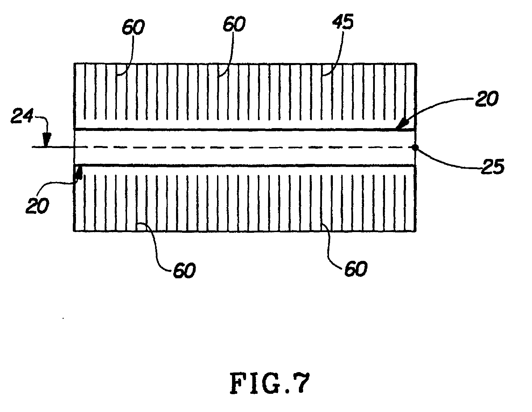

Figure 7 is a plan view of the top layer of the expandable member.

### DETAILED DESCRIPTION OF THE INVENTION

As used herein, the term "sanitary napkin" or "napkin" refers to an absorbent article which is worn by females adjacent to the pudendal region, generally external to the urogenital region, and which is intended to absorb and contain menstrual fluids and other vaginal discharges from the wearer's body (e.g., blood, menses, and urine). As used herein, the term "pudendal" refers to the externally visible female genitalia. It should be understood, however, that the present invention is also applicable to other feminine hygiene or catamenial pads such as pantiliners, or other absorbent articles such as incontinence pads, and the like. By the term "zone of menses insult" it is meant herein that area on the sanitary napkin most likely to consistently receive a menses discharge from a female wearer.

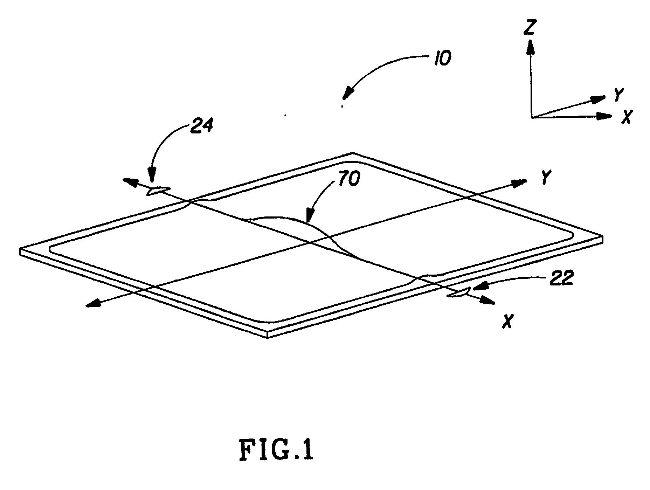

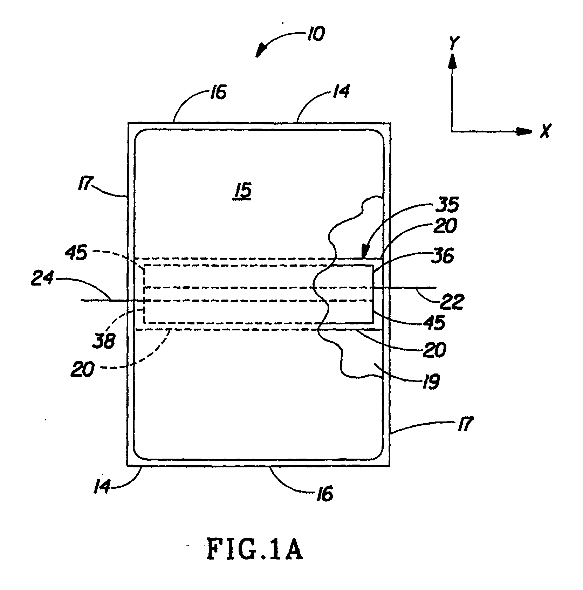

Figure 1 provides a perspective view of the sanitary napkin 10 having a hump 70 therein formed by a user prior and/or during the wear of the napkin 10. As is shown, the hump 70 resides substantially along the x-axis. Alternatively, the hump 70 may also substantially reside about the y-axis. Of course, the orientation of the first and second cinch members 22 and 24, respectively, must coincide positionally to operatively function with the expandable member 35 (Fig. 1A) to thereby create a raised hump 70. It is further noted herein that any number of humps 70 may be formed at various positions throughout the sanitary napkin 10. For example, one napkin 10 may comprise a hump 70 for menses collection while another hump 70, with separate cinch members therefor, resides on the napkin 10 for urine collection.

The hump 70 and the densification zone 70 may be as the same in one embodiment. This is because the expandable member 35 may be contracted and thus densified without a substantial hump protruding into the z-plane. Specifically, the densification zone 70 provides the expandable member 35 in its contracted state once the cinch members 22 and 24 have been pulled by a female user to contract the member 35. At such contraction of the member 35, a densified portion 70 is formed which substantially does not break into the z-plane. When the hump 70 is formed, the expandable member 35 has been contracted such that the expandable member 35 substantially elevates into the z-plane. Therefore, the expandable member 35, when contracted, will develop into one of two forms: 1) a densified zone 70 that does not substantially elevate into the z-plane or 2) a hump 70 which does substantially elevate into the z-plane.

The importance of a densification zone 70, of which there may be many such zones 70, is to provide densified zones of liquid collection, distribution and/or absorption. The zones 70 may, upon collection of liquids like urine, menses or any other types of vaginal discharges, distribute the liquid to other portions of the sanitary napkin 10. Elsewise or additionally, a densification zone may provide absorption of the aforesaid liquids, for example, right at the point of liquid impact.

A manufacturer may so design a sanitary napkin 10 herein that it contains one or more densification zones 70 that may be specifically placed at those known regions on a sanitary napkin to experience the greatest incidence of urine and/or menses insult. This, of course, is not meant to limit the placement or positioning of densification zones 70 for just urine or menses; i.e., other vaginal discharges may be designed for.

Figure 1A is a plan view of the sanitary napkin 10 of the present invention in its flat-out state with portions of the structure being cut-away to more clearly show the construction of the sanitary napkin 10 and with the portion of the sanitary napkin 10 which faces or contacts the wearer, oriented towards the viewer. The sanitary napkin 10 has two surfaces, a body-contacting or body facing surface or body surface and a garment surface. The body surface is that surface that lies adjacent to a user's pudendal region when the sanitary napkin 10 is worn. The garment surface is that surface positioned away from a user's pudendal region when the sanitary napkin 10 is worn. The sanitary napkin 10 is shown in Figure 1A as viewed from its body facing surface. The body facing surface is intended to be placed adjacent to the body of the wearer while the garment surface is on the opposite side and is intended to be placed adjacent to the wearer's undergarments when the sanitary napkin 10 is worn. The sanitary napkin 10 has two centerlines; i.e., a longitudinal centerline 15 and a transverse centerline 17. The term "longitudinal", as used herein, refers to a line, axis or direction in the plane of the sanitary napkin 10 that is generally aligned with (e.g., approximately parallel to) a vertical plane which bisects a standing wearer into left and right body halves when the sanitary napkin 10 is worn.

The terms "transverse" or "lateral" as used herein, are interchangeable, and refer to a line, axis or direction which lies within the plane of the sanitary napkin 10 that is generally perpendicular to the longitudinal direction. Figure 1A also shows that the sanitary napkin 10 has a periphery 14 which is defined by the outer edges of the sanitary napkin 10 in which the longitudinal edges are designated 17 and the end edges are designated 16.

The sanitary napkin 10 comprises a topsheet 15, a backsheet 18 positioned adjacent to and attached to the topsheet 15, and an absorbent core 19 positioned between the topsheet 15 and the backsheet 18. The topsheet 15 and backsheet 18 come together to form a common periphery 14 formed by end edges 16 and longitudinal edges 17.

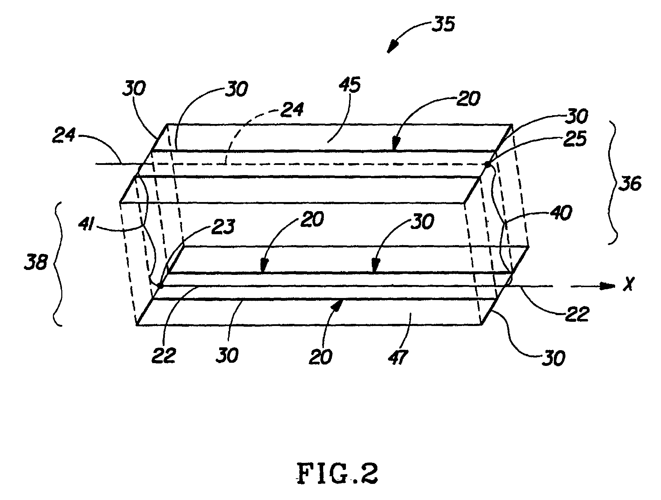

The sanitary napkin 10 further comprises an expandable member 35 having a first end 36 and a second end 38 positioned between the topsheet 15 and the backsheet 18. Also, the expandable member 35 comprises a pair of longitudinal edges 23. The expandable member 35 may or may not be a part of the absorbent core 19. Further, the expandable member 35 may reside below the absorbent core 19 and adjacent to the backsheet 18 or above the absorbent core 19 an adjacent to the topsheet 15. Preferably, the expandable member 35 will comprise at least two layers, as is shown in Figs. 2, 3, and 4. More specifically, the sanitary napkin 10 will preferably comprise an expandable member 35 having a top layer 45 and a bottom layer 47. (Figs. 2-4). In use, the end edges 36 and 38 and the longitudinal edges 33 of the multi-layered expandable member 35 line up with one another for attachment of layers along their aligned edges. Suitable materials for use for the top layer 45 or bottom layer 47 are nonwovens, sponge material, polyethylene, polypropylene, suede, vinyl, leather, any of several known polymeric materials in the art and combinations thereof.

The expandable member 35 may be fringed along its longitudinal edges. Fig. 9 shows a top plan view of the top layer 47 of the expandable member 35. As seen, fringes 60 line-up in a perpendicular orientation to the confining channel 20. The purpose of the fringes 60 is to provide greater surface area and bulkiness to the member 35. The fringes 60 shown in the top layer 47 correspond exactly to the fringes 60 (not shown) in the bottom layer 47 which is not shown. The fringes 60 most preferably consist of slits or cuts in the top layer 45 and the bottom layer 47. Such cutting can be done mechanically by a knife.

The expandable member 35 may be raised at various heights; e.g., from about 3 mm to about 50 mm. Furthermore, the expandable member 35, by virtue of its composition may be as stiff or as pliable as determined by a manufacturer. Preferably, once the expandable member 35 has been activated and thus raised by a female user to a specific position, it will hold that position substantially during the wear of the sanitary napkin 10. Also, preferably, the hump 70 of the sanitary napkin 10 is positioned to be within the zone of menses insult of a female user.

Additionally, the expandable member 35 will comprise cinch members attached thereto; i.e., first cinch member 22 and second cinch member 24. First cinch member 22 is attached to the bottom layer 47 at the connection point 23 which is positioned on the second end edge 38 of the expandable member 35. In like fashion, second cinch member 24 is attached to the top layer 45 at the connection point 25 which is along the first end 36 of the expandable member 35. This orientation is formed such that the cinch members 22 and 24 may be pulled into the direction opposite to the side of the expandable member 35 on which they are attached. It is further noted herein that the first cinch member 22 is preferably positioned adjacent to the top surface of the bottom layer 47 of the member 35. Also preferably, the second cinch member 24 is positioned adjacent to the bottom surface of the top layer 45 of the member 35. However, the cinch members 22 and 24 may also be attached to the member 35 at the points 23 and 25 by mechanical means (such as crimping, embossing, etc.), ultrasonic bonding, thermal bonding, or any other suitable means known in the art.

In practice each cinch member (22 and 24) will be pulled in opposite directions through the top layer 45 and the bottom layer 47 of the expandable member 35. More specifically, the first cinch member 22 is positioned above the bottom layer 47 and the second cinch member is positioned below the top layer 45. In this configuration, each cinch member is pulled through openings 40 and 41. The openings 40 and 41 are formed by free spaces between the top layer 45 and the bottom layer 47 that are not attached to one-another. It should be noted herein that Figs. 2-4 show exploded views of the expandable member 35. In practice, the top layer 45 and bottom layer 47 are attached to one-another about their shared periphery, which includes their end edges 16 and their longitudinal edges 17.

The expandable member 35 also comprises a confining channel 20 and connection lines 30. As shown in Fig. 1, the confining channel 20 extends in the direction of the x-axis from one longitudinal edge 17 to the other longitudinal edge 17. The confining channel 20 is a channel formed by creating a secure attachment along the connection lines 30 shown. The attachment is between the top layer 45 and the bottom layer 47. Between the connection lines 30 are portions of unattachment between the top layer 45 and the bottom layer 47 which make up the openings 40 and 41 of the expandable member 35. As is also shown, preferably, the connection lines 30 will extend along the first end 36 of the expandable member 35 and also along the second end 38 of the expandable member 35 to provide attachment along the ends 36 and 38 everywhere but at the openings 40 and 41. The attachments formed between the top layer 45 and the bottom layer 47, the confining channel 20 and the connection lines are formed from suitable adhesives known in the art for use with absorbent articles. For example, the known adhesives in the art for securing a topsheet to a backsheet in a diaper, sanitary napkin or like article are highly desirable for the attachments listed above. Adhesives which have been found to be satisfactory are manufactured by H. B. Fuller Company of St. Paul, Minnesota under the designation HL-1258 or H-2031. Other suitable bonding processes known in the art may also be used; e.g., ultrasonic bonding, thermal bonding, and others.

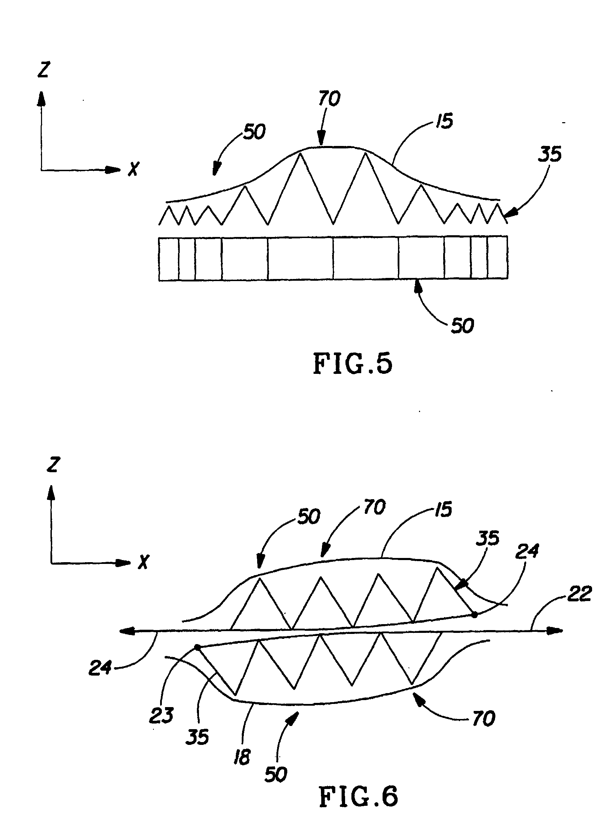

When the cinch members 22 and 24 are pulled through their respective openings 40 and 41, the ends 36 and 38 of the expandable member 35 are pulled closer together, thereby causing the mechanically expandable sanitary napkin 10 to elevate out of the x-y plane and into the z-plane, thus creating a hump 70. Such pulling of the cinch members 22 and 24 across the expandable member 35 forms a raised and puffed hump 70 which substantially breaks the x-y plane of the mechanically expandable sanitary napkin orientation. (See Figs. 5 and 6). Such expansion into the z-direction of a sanitary napkin by a female user provides improved fit to the user. Notably, a female user may expand the napkin 10 upward into and adjacent to her perineal grooves and labia. The labia, an organ suited to grasping, may then grasp the sanitary napkin's 10 hump 70 preferably throughout the wear of the sanitary napkin 10. The hump 70 may then adjacently intercept vaginal fluid such as menses from a female user. By the term "adjacently intercept" it is meant herein that the hump 70 because of its adjacent relationship to a user's perineum and labia may by direct contact thereto receive released vaginal fluids from a female user. This would, of course, apply equally as well in an urinary incontinent device as in a device formed primarily for menses collection provided that the hump 70 is properly positioned on the sanitary napkin 10. In fact, an embodiment is envisioned herein wherein a sanitary napkin 10 comprises at least two or more humps 70 that are mechanically operated by a user; one hump 70 constructed for light urinary incontinence and another hump 70 constructed for menses and other vaginal fluid collection.

In one embodiment herein, the hump 70 operates primarily as a fluid distribution mechanism. That is, the hump 70 will substantially not absorb vaginal fluids like menses but will readily collect and distribute the menses to other fluid absorbing portions of the sanitary napkin 10; e.g., the absorbent core 19. Such fluid distribution is performed by components in the expandable member 35 specifically designed for such fluid distribution. Such components include the use of inherently, hydrophobic fibers, polyethylene fibers, polypropylene fibers, capillary channel fibers, and cellulosic fibers treated with a hydrophobic agent thereon; this list is not meant to be exhaustive. In fact, any fibers which are hydrophobic or made to be hydrophobic and are known in the art to be suitable for the use in an absorbent article are envisioned for the expandable member 35.

Another manner of achieving fluid transfer is through the creation of a density gradient between the top layer 45 and the bottom layer 47. In such a density gradient envisioned, fluid would be drawn from the topsheet 15 down through the top and bottom layers (45 and 47) of the expandable member 35 and subsequently into the absorbent core 19. Obviously, such a density gradient is formed where the expandable member 35 resides above (or on top of) the absorbent core 19 and adjacent to the topsheet 15. U.S. Application Serial No. 08/791,094 entitled "Sanitary Napkin Comprising An Absorbent Core Having A Density Gradient" describes the aforementioned density gradient and is hereby incorporated by reference herein.

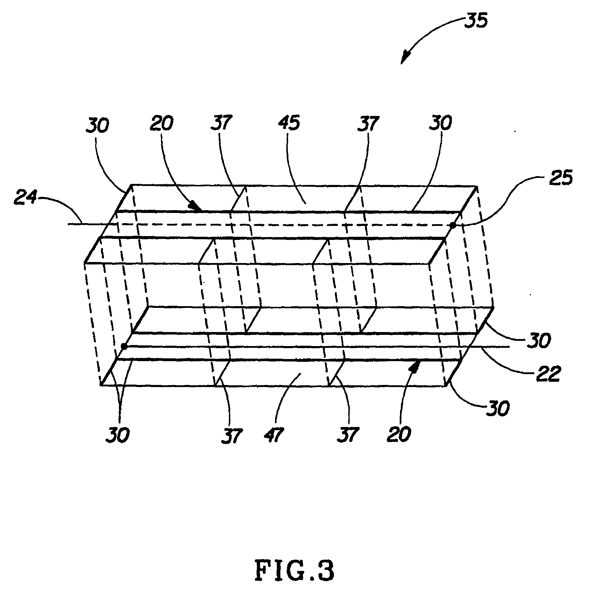

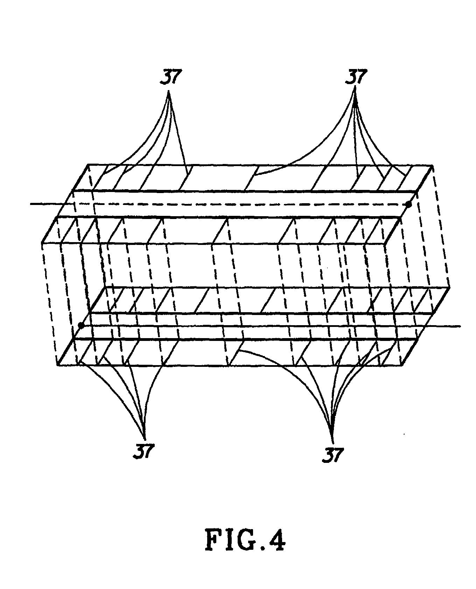

Figures 3 and 4 show alternative embodiments of the embodiment shown in Fig. 2. Fig. 3 additionally comprises crease lines 37 which are additional lines of attachment between the top layer 45 and the bottom layer 47 of the expandable member 35. The use of the crease lines 37 creates cinch profiles 50 (Figs. 5 and 6) whereby the expandable member 35 will cinch or hump in a prescribed fashion corresponding to the settings of the crease lines. For example, Fig. 5 shows a cinch profile made up of a crease line 37 pattern which causes the resultant cinch profile 50 of the expandable member 35. Furthermore, in a multi-layered member 35, this cinch profile 50 also indicates that the top layer 45 of the member 35 is more rigid than the bottom layer 47. When the top layer 45 and the bottom layer 47 comprise materials having differing rigidities, whichever layer is most flexible will be the layer that partially, nearly or substantially conforms to the more rigid layer. At this conformity, especially where it is the pronounced sort shown in Fig. 5, one layer of the expandable member 35 will be substantially elevated in the z-direction while the other layer either conforms substantially to the elevated layer; i.e., the less rigid layer elevates to conform with the humps or creases of the more rigid layer.

The crease lines 37 may be formed by adhesive such as that used to attach the top layer 45 and the bottom layer 47 of the expandable member 35. Additionally, the crease lines 37 may be formed from any suitable bonding process which will bind, i.e., attach, those portions of the top layer 45 and the bottom layer 47 shown in Figs. 3-5. Such bonding techniques include thermal bonding, ultrasonic bonding, crimping, embossing and any other suitable mechanical bonding technique known in the art. Furthermore, any known bonding technique in the art suitable for attaching top layer 45 and bottom layer 47 is hereby proscribed herein.

Obviously, such one-sided conformity is important where it is desired to create a sanitary napkin 10 that "puffs" or "humps" substantially in one direction. By the terms "puffs" or "humps" it is meant herein that the expandable member 35 will move out of the x and y planes and into the z-plane. However, Fig. 6 shows an embodiment wherein both layers of the member 35 expand out of the x and y planes and into the z-plane. Generally, this occurs when the multiple layers of the expandable member 35 are at least of approximately equal rigidity. This is also an important feature because for certain functions it may be desired to have a sanitary napkin 10 which comprises a two-sided hump 70.

In another embodiment herein, the expandable member 35 may be multi-layered. That is, the expandable member 35 may have additional layers attached to either the top layer 45 or the bottom layer 47 to add bulkiness or stiffness to the member 35. In one particular embodiment, a layer or layers may be positioned between the top layer 45 and the bottom layer 47 without any substantial interference with the attachments achieved by the confining channel 20. This intervening layer 100 (not shown) may be provide an increased rigidity to the member 35 such that when the member 35 is contracted by the cinch members 22 and 24, the intervening layer 100 helps to lock the member 35 in place. By the term "lock" it is meant herein that the member 35 will be caused to be substantially fixed in its contracted position. Such an intervening layer 100 may comprise a nonwoven, foam, polyethylene, polypropylene, a polymer scrim or any combination of the foregoing.

While the topsheet, the backsheet, and the absorbent core may be assembled in a variety of well known configurations (including so called "tube" products or side flap products), preferred sanitary napkin configurations are described generally in U.S. Patent No. 4,950,264, "Thin, Flexible Sanitary Napkin" issued to Osborn on August 21, 1990; U.S. Patent No. 4,425,130, "Compound Sanitary Napkin" issued to DesMarais on January 10, 1984; U.S. Patent No. 4,321,924, "Bordered Disposable Absorbent Article" issued to Ahr on March 30, 1982; U.S. Patent No. 4,589,876, "Shaped Sanitary Napkin With Flaps" issued to Van Tilburg on August 18, 1987. Each of these patents are hereby incorporated herein by reference. Figure 1 shows a preferred embodiment of the sanitary napkin 10 in which the topsheet 24 and the backsheet 26 have length and width dimensions generally larger than those of the absorbent core 28. The topsheet 24 and the backsheet 26 can extend beyond the edges of the absorbent core 28 to thereby form not only portions of the periphery but also side flaps.

The absorbent core 19 may be any absorbent means which is capable of absorbing or retaining liquids (e.g., menses and/or urine). As shown in Figure 1A, the absorbent core 19 has a body surface, a garment surface, side edges, and pad edges. The absorbent core 19 may be manufactured in a wide variety of sizes and shapes (e.g., rectangular, oval, hourglass, dog bone, asymmetric, etc.) and from a wide variety of liquid-absorbent materials commonly used in sanitary napkins and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. The configuration and construction of the absorbent core may also be varied (e.g., the absorbent core may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core should, however, be compatible with the design loading and the intended use of the sanitary napkin. Further, the size and absorbent capacity of the absorbent core may be varied to accommodate different uses such as incontinence pads, pantiliners, regular sanitary napkins, or overnight sanitary napkins.

Exemplary absorbent structures for use as the absorbent core of the present invention are described in U.S. Patent No. 4,950,264 entitled "Thin, Flexible Sanitary Napkin" issued to Osborn on August 21, 1990; U.S. Patent No. 4,610,678 entitled "High-Density Absorbent Structures" issued to Weisman et al. on September 9, 1986; U.S. Patent No. 4,834,735 entitled "High Density Absorbent Members Having Lower Density and Lower Basis Weight Acquisition Zones", issued to Alemany et al. on May 30, 1989; and European Patent Application No. 0 198 683, The Procter & Gamble Company, published October 22, 1986 in the name of Duenk, et al. Each of these patents are incorporated herein by reference.

The backsheet 18 and the topsheet 15 are positioned adjacent the garment surface and the body surface, respectively, of the absorbent core 19 and are preferably joined thereto and to each other by attachment means (not shown) such as those well known in the art. For example, the backsheet 18 and/or the topsheet 15 may be secured to the absorbent core 19 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent No. 4,573,986 entitled "Disposable Waste-Containment Garment", which issued to Minetola, et al. on March 4, 1986, and which is incorporated herein by reference. An exemplary attachment means of an open pattern network of filaments comprises several lines of adhesive filaments swirled into a spiral pattern such as illustrated by the apparatus and method shown in U.S. Patent No. 3,911,173 issued to Sprague, Jr. on October 7, 1975; U.S. Patent No. 4,785,996 issued to Zieker, et al. on November 22, 1978; and U.S. Patent No. 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet 18 is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet 18 prevents the exudates absorbed and contained in the absorbent core 19 from wetting articles which contact the sanitary napkin 10 such as pants, pajamas and undergarments. The backsheet 18 may thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet 18 may permit vapors to escape from the absorbent core 19 (i.e., breathable) while still preventing exudates from passing through the backsheet 18.

The topsheet 15 is compliant, soft feeling, and non-irritating to the wearer's skin. Further, the topsheet 24 is liquid pervious permitting liquids (e.g., menses and/or urine) to readily penetrate through its thickness. A suitable topsheet 15 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. A preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3.929,135, entitled "Absorptive Structures Having Tapered Capillaries", which issued to Thompson on December 30, 1975; U.S. Patent No. 4,324,246 entitled "Disposable Absorbent Article Having A Stain Resistant Topsheet", which issued to Mullane, et al. on April 13, 1982; U.S. Patent No. 4,342,314 entitled "Resilient Plastic Web Exhibiting Fiber-Like Properties", which issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", which issued to Ahr et al. on July 31, 1984; and U.S. Patent No. 5,006,394 "Multilayer Polymeric Film" issued to Baird on April 9, 1991. Each of these patents are incorporated herein by reference. The preferred topsheet for the present invention is the formed film described in one or more of the above patents and marketed on sanitary napkins by The Procter & Gamble Company of Cincinnati, Ohio as "DRI-WEAVE".

In a preferred embodiment of the present invention, the body surface of the formed film topsheet is hydrophilic so as to help liquids to transfer through the topsheet faster than if the body surface was not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al., which is incorporated herein by reference. Alternatively, the body surface of the topsheet can be made hydrophilic by treating it with a surfactant such as is described in the above referenced U.S. Patent No. 4,950,254 issued to Osborn, and is incorporated herein by reference.

In use, the sanitary napkin 10 can be held in place by any support means or attachment means (not shown) well-known for such purposes. Preferably, the sanitary napkin is placed in the user's undergarment or panty and secured thereto by a fastener such as an adhesive. The adhesive provides a means for securing the sanitary napkin in the crotch portion of the panty. Thus, a portion or all of the outer surface of the backsheet 18 is coated with adhesive. Any adhesive or glue used in the art for such purposes can be used for the adhesive herein, with pressure-sensitive adhesives being preferred. Suitable adhesives are Century A-305-IV manufactured by the Century Adhesives Corporation of Columbus, Ohio; and Instant Lock 34-2823 manufactured by the National Starch and Chemical Company of Bridgewater, NJ. Suitable adhesive fasteners are also described in U.S. Patent No. 4,917,697. Before the sanitary napkin is placed in use, the pressure-sensitive adhesive is typically covered with a removable release liner in order to keep the adhesive from drying out or adhering to a surface other than the crotch portion of the panty prior to use. Suitable release liners are also described in the above-referenced U.S. Patent No. 4,917,697. Any commercially available release liners commonly used for such purposes can be utilized herein. Non-limiting examples of suitable release liners are BL30MG-A Silox E1/0 and BL30MG-A Silox 4P/O both of which are manufactured by the Akrosil Corporation of Menasha, WI. The sanitary napkin 10 of the present invention is used by removing the release liner and thereafter placing the sanitary napkin in a panty so that the adhesive contacts the panty. The adhesive maintains the sanitary napkin in its position within the panty during use.

The sanitary napkin 10 may comprise two flaps, each of which is adjacent to and extend laterally from the side edges 17 of the sanitary napkin 10. The flaps are configured to drape over the edges of the wearer's panties in the crotch region so that the flaps are disposed between the edges of the wearer's panties and thighs. The flaps serve at least two purposes. First, the flaps help serve to prevent soiling of the wearer's body and panties by menstrual fluid, preferably by forming a double wall barrier along the edges of the panty. Secondly, the flaps are preferably provided with attachment means on their garment surface so that the flaps can be folded back under the panty and attached to the garment facing side of the panty. In this way, the flaps serve to keep the sanitary napkin 10 properly positioned in the panty. The flaps can be constructed of various materials including materials similar to the topsheet, backsheet, tissue, or combination of these materials. Further, the flaps may be a separate element attached to the main body of the napkin 10 or can comprise extensions of the topsheet and backsheet (i.e., unitary). A number of sanitary napkins having flaps suitable or adaptable for use with the sanitary napkins of the present invention are disclosed in U.S. Patent No. 4,687,478 entitled "Shaped Sanitary Napkin With Flaps", which issued to Van Tilburg on August 18, 1987; U.S. Patent No. 4,589,876 entitled "Sanitary Napkin", which issued to Van Tilburg on May 20, 1986; and U.S. Patent No. 4,608,047, entitled "Sanitary Napkin Attachment Means", which issued to Mattingly on August 26, 1986. Each of these patents are incorporated herein by reference.

In a preferred embodiment herein, the flaps comprise an attachment means having hooks. The hooks may have a mating element known as loops, but more preferably, the hooks will themselves be attachable to the nonwoven surface of the flaps. Exemplary attachment systems comprising hook and loop fastening materials are disclosed in U.S. Patent No. 4,869,724 issued to Scripps on September 26, 1989. Attachment systems utilizing mechanical closure elements are also disclosed in U.S. Patent No. 4,846,815 issued to Scripps on July 11, 1989; and U.S. Patent No. 4,894,060 issued to Nestegard on January 16, 1990. Attachment systems having combination adhesive/mechanical closure elements are described in U.S. Patent No. 4,946,527 issued to Battrell on August 7, 1990. Each of these patents are incorporated herein by reference.

In a preferred embodiment of the present invention, an acquisition layer(s) (not shown) may be positioned between the topsheet and the absorbent core, and either above or below the expandable member. The acquisition layer may serve several functions including improving wicking of exudates over and into the absorbent core. There are several reasons why the improved wicking of exudates is important, including providing a more even distribution of the exudates throughout the absorbent core and allowing the sanitary napkin 10 to be made relatively thin. (The wicking referred to herein may encompass the transportation of liquids in one, two or all directions (i.e., in the x-y plane and/or in the z-direction). The acquisition layer may be comprised of several different materials including nonwoven or woven webs of synthetic fibers including polyester, polypropylene, or polyethylene; natural fibers including cotton or cellulose; blends of such fibers; or any equivalent materials or combinations of materials. Additionally, crimped, synthetic fibers of the aforementioned as well as chemically stiffened, cellulosic fibers may be used. Examples of sanitary napkins having an acquisition layer and a topsheet are more fully described in U.S. Patent No. 4,950,264 issued to Osborn and U.S. Patent Application Serial No. 07/810,774, "Absorbent Article Having Fused Layers", filed December 17, 1991 in the names of Cree, et al. Each of these references are incorporated herein by reference. In a preferred embodiment, the acquisition layer may be joined with the topsheet by any of the conventional means for joining webs together, most preferably by fusion bonds as is more fully described in the above-referenced Cree application.

Preferably, once the cinch members 22 and 24 are pulled or extended through openings 40 and 41, the cinch members will remain stationary such that the expanded structure of the expandable member 35 will remain in its expanded configuration. To these ends, one embodiment herein contemplates providing the cinch members with tape tabs and/or hooks and loops (i.e., fastening systems) so that when the cinch members 22 and 24 are pulled, they may either be brought around to either the topsheet 15 or backsheet 18 of the mechanically expandable sanitary napkin 10 and be secured thereto or secured to one-another. If, for example, the backsheet 18 comprises a nonwoven layer, the ends of the cinch members 22 and 24 may each have attached thereon a tab 7 (Fig. 1) comprising hooks which can engage the nonwoven backsheet 18 and remain fixed thereto. Alternatively, if the backsheet comprises polymer material, the ends of the cinch members 22 and 24 may likewise comprise tabs 7 having tape thereon that readily adhere to the backsheet 18. Preferably, such tape tabs would also be readily releasable from the backsheet 18. These cinch member attachments devices notwithstanding, preferably the cinch members 22 and 24 are constructed such that when they are pulled, the expandable member 35 remains in a cinched position by virtue of the rigidity of one or more of the layers (top 45 or bottom 47) that make-up the expandable member 35.

Exemplary fastening systems are disclosed in U.S. Patent No. 4,846,815 entitled "Disposable Diaper Having An Improved Fastening Device" issued to Scripps on July 11, 1989; U.S. Patent No. 4,894,060 entitled "Disposable Diaper With Improved Hook Fastener Portion" issued to Nestegard on January 16, 1990; U.S. Patent No. 4,946,527 entitled "Pressure-Sensitive Adhesive Fastener And Method of Making Same" issued to Battrell on August 7, 1990; U.S. Patent No. 3,848,594 entitled "Tape Fastening System for Disposable Diaper" issued to Buell on November 19, 1974; U.S. Patent No. 4,662,875 entitled "Absorbent Article" issued to Hirotsu et al. on May 5, 1987; and the herein before referenced U.S. Patent Application No. 07/715,152; each of which is incorporated herein by reference. Exemplary fastening systems comprising mechanical fastening components (i.e., hooks and loops) are described in U.S. Patent No. 5,058,247 entitled "Mechanical Fastening Prong" issued to Thomas October 22, 1991; U.S. Patent No. 4,869,724 entitled "Mechanical Fastening Systems With Adhesive Tape Disposal Means For Disposal of Absorbent Articles" issued to Scripps on September 26, 1989; and U.S. Patent No. 4,846,815 entitled "Disposable Diaper Having an Improved Fastening Device" issued to Scripps on July 11, 1989. An example of a fastening system having combination mechanical/adhesive fasteners is described in U.S. Patent No. 4,946,527 entitled "Pressure-Sensitive Adhesive Fastener and Method of Making Same" issued to Battrell on August 7, 1990. Each of these patents are incorporated herein by reference.

## Claims

1. A sanitary napkin (10) having a center, a menses impact zone, a topsheet (15), a backsheet (18) attached to the topsheet (15), an absorbent core (19) positioned between the topsheet (15) and the backsheet (18), **characterized in that**:
an expandable member (35) having a first end (36), a second end (38), and a pair of longitudinal edges (23) is positioned between the topsheet (15) and the backsheet (18), and at least a pair of cinch members (22, 24) are attached to the expandable member (35), one said cinch member (22) being attached at the first end of the expandable member (35), the other said cinch member (24) being attached to the second end of the expandable member (35), and
each said cinch member (22, 24) extends out between the topsheet (15) and the backsheet (18) such that when the cinch members (22, 24) are pulled oppositely to one-another, the ends of the expandable member (35) pulled toward together to form a densified zone in the sanitary napkin.

2. The sanitary napkin of Claim 2 wherein the expandable member (35) forms a densified zone which does not substantially enter into the z-plane.

3. The sanitary napkin of Claim 1 wherein each said cinch member (22, 24) extends out between the topsheet (15) and the backsheet (18) such that when the cinch members (22, 24) are pulled oppositely to one-another, the ends of the expandable member (35) are pulled together to form a raised hump (70) in the sanitary napkin that extends into the z-plane.

4. The sanitary napkin of Claim 3 wherein the labia of a female user grasps the hump (70) of the sanitary napkin substantially throughout the wear of the sanitary napkin.

5. The sanitary napkin of Claim 3 wherein the hump (70) operates substantially as a fluid distribution mechanism or operates substantially as a fluid absorption mechanism.

6. The sanitary napkin of Claims 1 or 3 wherein a confining channel (20) houses the expandable member (35) and the cinch members (22, 24) and confines the relative motion of the expandable member (35) and the cinch members (22, 24) in a prescribed direction.

7. The sanitary napkin of Claims 1 or 3 wherein multiple densification zones or multiple humps (70) are formed within the sanitary napkin.

8. The sanitary napkin of Claim 3 wherein the hump (70) extends in one direction or two directions into the z-plane.

9. The sanitary napkin of Claim 1 wherein the densification zone or the hump (70) of the sanitary napkin is positioned to be within the menses impact zone of a female user.

10. The sanitary napkin of Claim 1 wherein the expandable member (35) comprises crease lines, each said crease line being oriented perpendicularly from the confining channel and extending from one side of the confining channel to the longitudinal edge of the expandable member.

11. The sanitary napkin of Claim 1 wherein the first end (36), the second (38) and the longitudinal edges (23) of the expandable member (35) are fringed.

12. The sanitary napkin of Claims 1 or 3 wherein the cinch members (22, 24) will remain stationary after the expandable member (35) has been expanded.

13. The sanitary napkin of Claims 1 or 3 wherein the cinch members (22, 24) comprise attachment devices (7) to attach to the backsheet (18) of the sanitary napkin to thereby make the expandable member (35) stationary and preferably wherein the attachment devices comprise at least one device selected from the group consisting of tape tabs, hooks, loops or a combination thereof.

## Patentansprüche

1. Damenbinde (10) mit einem Zentrum, einer Auftreffzone für Menstruationsfluide, einer Oberschicht (15), einer an der Oberschicht (15) angebrachten Unterschicht (18), einem zwischen der Oberschicht (15) und der Unterschicht (18) positionierten absorbierenden Kern (19), **dadurch gekennzeichnet, daß**:
ein expandierbares Element (35) mit einem ersten Ende (35), einem zweiten Ende (38) und einem paar Längsrändern (23) zwischen der oberen Schicht (15) und der unteren Schicht (18) positioniert ist, und wenigstens ein Paar Einziehelemente (22. 24) an dem expandierbaren Element (35) angebracht ist, wobei das eine Einziehelement (22) an dem ersten Ende des expandierbaren Elelments (35) angebracht ist, das andere Einziehelement (24) an dem zweiten Ende des expandierbaren Elements (35) angebracht ist; und
jedes Einziehelement (22, 24) sich zwischen der Oberschicht (15) und der Unterschicht (18) derart erstreckt, daß, wenn die Einziehelemente (23, 24) einander entgegengesetzt gezogen werden, die Enden des expandierbaren Elements (35) zueinander hingezogen werden, um eine verdichtete Zone in der Damenbinde zu bilden.

2. Damenbinde nach Anspruch 2, in welcher das expandierbare Element (35) eine verdichtete Zone bildet, welche nicht wesentlich in die z-Ebene eintritt.

3. Damenbinde nach Anspruch 1, in welcher sich jedes Einziehelement (22, 24) zwischen der Oberschicht (15) und der Unterschicht (18) derart erstreckt, daß wenn die Einziehelemente (22, 24) einander entgegengesetzt gezogen werden, die Enden des expandierbaren Elements (35) zueinander hingezogen werden, um einen erhabenen Buckel (70) in der Damenbinde zu bilden, der sich in die z-Ebene erstreckt.

4. Damenbinde nach Anspruch 3, in welcher die Labia einer Benutzerin den Bukkel (70) der Damenbinde im wesentlichen während der ganzen Tragezeit der Damenbinde angreift.

5. Damenbinde nach Anspruch 3, in welcher der Buckel (70) im wesentlichen als ein Fluid- Verteilungsmechanismus arbeitet oder im wesentlichen als ein Fluid-Absorptionsmechanismus arbeitet.

6. Damenbinde nach Anspruch 1 oder 3, in welcher ein Begrenzungskanal (20) das expandierbare Element (35) und die Einziehelemente (22,24) aufnimmt und die Relativbewegung des expandierbaren Elements (35) und der Einziehelemente (22, 24) in der vorbeschriebenen Richtung begrenzt.

7. Damenbinde nach den Ansprüchen 1 oder 3, in welcher mehrere Verdichtungszonen oder mehrere Buckel (70) innerhalb der Damenbinde ausgebildet sind.

8. Damenbinde nach Anspruch 3, in welcher der Buckel (70) sich in einer Richtung oder in zwei Richtungen in der z-Ebene erstreckt.

9. Damenbinde nach Anspruch 1, in welcher die Verdichtungszone oder der Bukkel (70) der Damenbinde so positioniert ist, daß sich dieser innerhalb der Auftreffzone von Menstruationsfluiden einer Benutzerin befindet.

10. Damenbinde nach Anspruch 1, in welcher das expandierbare Element (35) Falzlinien umfaßt, wobei jede Falzlinie rechtwinklig zum Begrenzungskanal orientiert ist und sich von einer Seite des Begrenzungskanals zu dem Längsrand des expandierbaren Elements erstreckt.

11. Damenbinde nach Anspruch 1, in welcher das erste Ende (36), das zweite (38) und der Längsrand (23) des expandierbaren Elements (35) gefranst sind.

12. Damenbinde nach den Ansprüchen 1 oder 3, in welcher die Einziehelemente (22, 24) stationär bleiben, nachdem das expandierbare Element (35) expandiert wurde.

13. Damenbinde nach den Ansprüchen 1 oder 3, in welcher die Einziehelemente (22, 24) Anbringungseinrichtungen (7) aufweisen, um diese an der Unterschicht (18) der Damenbinde anzubringen, um dadurch das expandierbare Element (35) stationär zu machen, und in welcher Vorzugsweise die Anbringungseinrichtungen wenigstens eine Einrichtung aufweisen, ausgewählt aus der Gruppe bestehend aus Bandstreifen, Haken, Schlingen oder eine Kombination davon.

## Revendications

1. Serviette hygiénique (10) ayant un centre, une zone d'impact des menstrues, une feuille de dessus (15), une feuille de fond (18) fixée à la feuille de dessus (15), une âme absorbante (19) placée entre la feuille de dessus (15) et la feuille de fond (18), **caractérisée en ce que** :
un élément dilatable (35) ayant une première extrémité (36), une deuxième extrémité (38) et une paire de bords longitudinaux (23) est situé entre la feuille de dessus (15) et la feuille de fond (18) et au moins une paire d'éléments de serrage (22, 24) est fixée à l'élément dilatable (35), l'un desdits éléments de serrage (22) étant fixé à la première extrémité de l'élément dilatable (35) et l'autre desdits éléments de serrage (24) étant fixé à la deuxième extrémité de l'élément dilatable (35), et
chaque élément de serrage (22, 24) s'étend entre la feuille de dessus (15) et la feuille de fond (18) si bien que lorsque les éléments de serrage (22, 24) sont tirés de façon opposée l'un à l'autre, les extrémités de l'élément dilatable (35) sont tirées conjointement pour former une zone densifiée dans la serviette hygiénique.

2. Serviette hygiénique selon la revendication 1, dans laquelle les éléments dilatables (35) forment une zone densifiée qui ne pénètre pas essentiellement dans le plan z.

3. Serviette hygiénique selon la revendication 1, dans laquelle chaque élément de serrage (22, 24) s'étend entre la feuille de dessus (15) et la feuille de fond (18) si bien que lorsque les éléments de serrage (22, 24) sont tirés à l'opposé l'un de l'autre, les extrémités de l'élément dilatable (35) sont tirées conjointement pour former une bosse relevée (70) dans la serviette hygiénique qui s'étend dans le plan z.

4. Serviette hygiénique selon la revendication 3, dans laquelle la lèvre d'une utilisatrice saisit la bosse (70) de la serviette hygiénique essentiellement pendant le port de la serviette hygiénique.

5. Serviette hygiénique selon la revendication 3, dans laquelle la bosse (70) agit essentiellement comme un mécanisme de répartition de fluide ou agit essentiellement comme un mécanisme d'absorption de fluide.

6. Serviette hygiénique selon la revendication 1 ou 3, dans laquelle un canal de confinement (20) loge l'élément dilatable (35) et les éléments de serrage (22, 24) et confine le mouvement relatif de l'élément dilatable (35) et des éléments de serrage (22, 24) dans une direction prescrite.

7. Serviette hygiénique selon la revendication 1 ou 3, dans laquelle des zones de densification multiples ou des bosses multiples (70) sont formées dans la serviette hygiénique.

8. Serviette hygiénique selon la revendication 3, dans laquelle la bosse (70) s'étend dans une direction ou deux directions dans le plan z.

9. Serviette hygiénique selon la revendication 1, dans laquelle la zone de densification ou la bosse (70) de la serviette hygiénique est disposée pour se situer dans la zone d'impact des menstrues d'une utilisatrice.

10. Serviette hygiénique selon la revendication 1, dans laquelle l'élément dilatable (35) comprend des lignes de pli, chaque ligne de pli étant orientée perpendiculairement à partir du canal de confinement et s'étendant depuis un côté du canal de confinement au bord longitudinal de l'élément dilatable.

11. Serviette hygiénique selon la revendication 1, dans laquelle la première extrémité (36), la deuxième extrémité (38) et les bords longitudinaux (23) de l'élément dilatable (35) sont frangés.

12. Serviette hygiénique selon la revendication 1 ou 3, dans laquelle les éléments de serrage (22, 24) restent fixes après que l'élément dilatable (35) ait été dilaté.

13. Serviette hygiénique selon la revendication 1 ou 3, dans laquelle les éléments de serrage (22, 24) comportent des dispositifs de fixation (7) pour la fixation à la feuille de fond (18) de la serviette hygiénique afin de rendre l'élément dilatable (35) fixe et, de préférence, dans laquelle les dispositifs de fixation comprennent au moins un dispositif choisi parmi le groupe comprenant un ruban, des pattes, des crochets, des boucles ou une combinaison de ceux-ci.
